(19) Europäisches Patentamt
European Patent Office
Office européen des brevets

(11) Veröffentlichungsnummer: **0 043 537**
**A1**

(12) # EUROPÄISCHE PATENTANMELDUNG

(21) Anmeldenummer: **81105054.1**

(22) Anmeldetag: **30.06.81**

(51) Int. Cl.³: **C 09 B 49/10**, C 09 B 53/02, C 07 D 209/88, D 06 P 1/30

(30) Priorität: **05.07.80 DE 3025549**

(43) Veröffentlichungstag der Anmeldung: **13.01.82 Patentblatt 82/2**

(84) Benannte Vertragsstaaten: **DE FR GB IT**

(71) Anmelder: **CASSELLA Aktiengesellschaft, Hanauer Landstrasse 526, D-6000 Frankfurt am Main 61 (DE)**

(72) Erfinder: **Piesch, Steffen, Dr., An der Heide 32, D-6370 Oberursel/Taunus (DE)**
Erfinder: **Weidemüller, Wolf, Dr., Nordring 101, D-6000 Frankfurt am Main (DE)**

(74) Vertreter: **Urbach, Hans-Georg, Dr. et al, Hanauer Landstrasse 526, D-6000 Frankfurt am Main 61 (DE)**

(54) **Schwefelfarbstoffe, ihre Herstellung und Verwendung sowie Zwischenprodukte zur Herstellung der Schwefelfarbstoffe.**

(57) Leuco-indophenole bzw. -indamine der Formel I

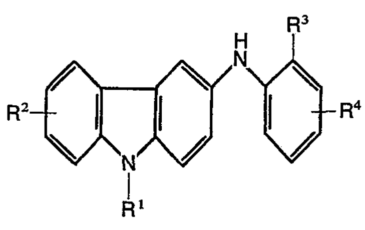

(I)

wobei $R^1$ Wasserstoff oder Alkyl mit 1 bis 8 C-Atomen, $R^2$ Wasserstoff oder Halogen, $R^3$ OH, $NH_2$ oder NHCO-Alkyl mit 1 bis 4 C-Atomen im Alkylrest und $R^4$ Wasserstoff, Halogen oder Alkyl mit 1 bis 4 C-Atomen bedeuten, die durch Umsetzung von 3-Aminocarbazolen der Formel V

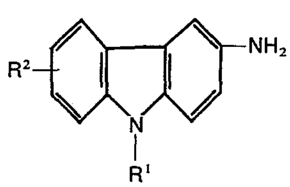

(V)

mit Verbindungen der Formel VI

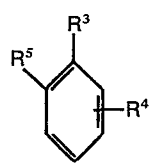

(VI)

unter Ausschluß von Sauerstoff bei Temperaturen von 150 bis 300 °C erhalten werden, dienen zur Herstellung von Schwefelfarbstoffen.

EP 0 043 537 A1

Schwefelfarbstoffe, ihre Herstellung und Verwendung sowie Zwischenprodukte zur Herstellung der Schwefelfarbstoffe

Die Erfindung betrifft neue Schwefelfarbstoffe der Carbazolreihe, die durch Schwefeln neuer Leuco-indophenole bzw. -indamine der Formel I

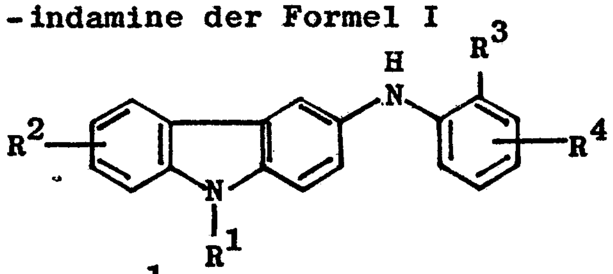

(I)

wobei $R^1$ Wasserstoff oder Alkyl mit 1 bis 8 C-Atomen, $R^2$ Wasserstoff oder Halogen, $R^3$ OH, $NH_2$ oder NHCO-Alkyl mit 1 bis 4 C-Atomen im Alkylrest und $R^4$ Wasserstoff, Halogen oder Alkyl mit 1 bis 4 C-Atomen bedeuten, nach an sich bekannten Verfahren herstellbar sind, ihre Verwendung zum Färben von Zellulose und Polyamid sowie die zur Herstellung der neuen Farbstoffe benötigten neuen Leuco-indophenole und -indamine der Formel I.

Es ist bekannt, durch Umsetzung geeigneter organischer Verbindungen mit Schwefel und gegebenenfalls Alkalisulfiden oder Alkalipolysulfiden bei erhöhter Temperatur Schwefelfarbstoffe herzustellen. Eine Übersicht über die wichtigsten Gruppen bisher bekannter, geeigneter Ausgangsmaterialien findet sich beispielsweise in Kirk-Othmer "Encyclopedia of Chemical Technologie", 2. Auflage, Band 19, Seiten 425 bis 435. Gemäß der großen technischen Bedeutung der Schwefelfarbstoffe existiert eine große Anzahl von Veröffentlichungen, die sowohl die allgemeinen Grundzüge der verschiedenen Schwefelungsverfahren, wie Backschmelze und Kochschmelze, betreffen, als auch einzelnen Gruppen geeigneter Ausgangsmaterialien und Schwefelfarbstoffen gewidmet sind. In diesem Zusammenhang sind insbesondere die Monographien von O. Lange "Die Schwefelfarbstoffe, ihre Herstellung und Verwendung", 2. Ausgabe, Spamer, Leipzig (1925) sowie K. Venkataraman "The Chemistry of Synthetic Dyes and Pigments", Band 2, Academic Press, Inc., New York (1952), Kapitel 35, von Interesse.

Aus einer ganzen Reihe von Patenten, z.B. der deutschen Patentschrift 218 371, dem britischen Patent 2918/09, der US-Patentschrift 919 572 sowie den betreffenden Abdrucken in "Friedländer", Band 10, Seiten 256, 258, 71 bis 74 und 75 bis 81, ist es bekannt, Leuco-Carbazolindophenole der Formel II

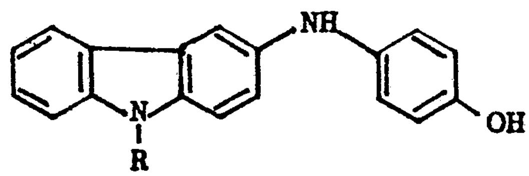

(II)

worin R Wasserstoff oder Ethyl ist, zu den blauen Schwefelfarbstoffen C.I. Sulfur Blue 42 und 43 zu schwefeln.

Weitere Derivate dieses Ausgangsmaterials sind bis-lang nicht zu Schwefelfarbstoffen verarbeitet worden, obwohl die oben genannten C.I.Sulfur Blue 42 und 43 ausgezeichnete Eigenschaften haben und bereits seit 70 Jahren bekannt sind. Zieht man in Betracht, daß Farbstoffe dieses Typs auch in anderen als blauen Nuancen von größtem Interesse gewesen wären, so erkennt man, daß alle Anstrengungen, das Gebiet der Carbazolindophenol-Schwefelfarbstoffe auszubauen, bisher gescheitert sind.

Ein wesentliches Moment für diese Mißerfolge dürfte darin zu erblicken sein, daß bislang keine ausreichend variablen Verfahren zur Herstellung der Ausgangsmaterialien der Formel II bekannt waren. Die Verbindungen der Formel II wurden durch Reduktion der entsprechenden Carbazolindophenole (Carbazol-3-iminochinone bzw. Benzochinon-(1,4)-mono-carbazolyl-(3)-imide) hergestellt, für deren Herstellung bis jetzt im wesentlichen zwei Verfahren bekannt geworden sind:

1. Durch Kondensation von Carbazol bzw. von N-substituierten Carbazolen mit p-Nitrosophenol in kalter, hochprozentiger Schwefelsäure unter Kühlung (FR-PS 457 535 , deutsche Reichspatentschriften 218371, 224 951 und 230 119).

2. Durch Umsetzung von Carbazol und seiner Derivate mit p-Aminophenol in konzentrierter Schwefelsäure unter Kühlung

in Gegenwart eines Oxidationsmittels, wie Mangandioxid (FR-PS 457 535).

Die anschließende Reduktion der Carbazolindophenole zu den Leucoverbindungen erfolgt im allgemeinen in verdünnter Säure mit Eisen.

In O.Lange "Die Zwischenprodukte der Teerfarbenfabrikation", Leipzig, 1920, wird auf Seite 315 auf die deutsche Patentanmeldung A 25 495, Klasse 12 P, vom 24.2.1914 hingewiesen, in der Herstellung der Indophenole und ihrer Leuco-Verbindungen aus 3-Aminocarbazol oder seinen N-substituierten Derivaten durch Kondensation mit Hydrochinon in Gegenwart wasserentziehender Mittel unter Vermeidung der Oxidation beschrieben sein soll. Die Anmeldung wurde nicht veröffentlicht, so daß Details dieses Verfahrens nicht bekannt geworden sind. Bei der Durchführung der Reaktion in Gegenwart der damals bekannten wasserentziehenden Mittel, nämlich konzentrierter Schwefelsäure oder Zinkchlorid, ist die Aufarbeitung durch Wasser zwingend notwendig, und man erhält das Leuco-indophenol nur in geringer Ausbeute neben einer Reihe von unerwünschten Nebenprodukten. Die Reinigung der so erhaltenen Leuco-indophenole gestaltet sich schwierig und schließt die Anwendung dieses Verfahrens für die technische Herstellung der gewünschten Produkte aus. Auch die oben genannten bekannten Herstellungsverfahren liefern die gewünschten Indophenole bzw. Leuco-indophenole nicht in der gewünschten Ausbeute und Reinheit und konnten im Laufe der langen Zeit, seit der sie bekannt wurden, nicht soweit verbessert werden, daß sie technisch durchgeführt werden. Als optimales bekanntes Verfahren hat sich bisher in der Technik das oben unter 1. genannte Kondensationsverfahren behauptet, bei dem zunächst p-Nitrosophenol mit dem Carbazolderivat kondensiert wird und das erhaltene chinoide Zwischenprodukt des Indophenols anschließend in der Regel reduziert werden muß, da das chinoide Produkt besonders instabil ist. Dieses technische Verfahren ist auf die Herstellung von 3-(4-Hydroxyphenylamino)-carbazol und seinem 9-Ethylderivat beschränkt; es ist nicht gelungen, es auf die Herstellung von anderen

Derivaten dieser Verbindung auszudehnen.

Nach einem älteren Vorschlag (deutsche Patentanmeldung P 29 00 441.9) können diese Verbindungen jedoch auch dadurch erhalten werden, daß ein 3-Aminocarbazol der allgemeinen Formel III

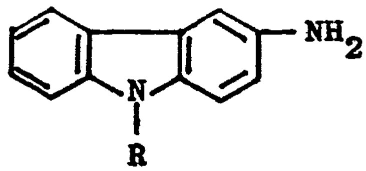 (III)

mit p-Hydrochinon oder p-Aminophenol oder mit einem Gemisch aus p-Hydrochinon und p-Aminophenol in mindestens stöchiometrischen Mengen unter Ausschluß von Sauerstoff auf Temperaturen von 150 bis 300°C erhitzt wird.

Nach einem weiteren Vorschlag (deutsche Patentanmeldung P 30 13 994.7) können Schwefelfarbstoffe auch durch Schwefeln von Leucocarbazolindophenolen bzw. -indaminen der Formel IV

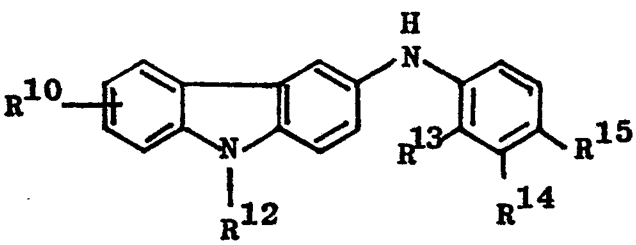 (IV)

erhalten werden, wobei $R^{10}$ Wasserstoff oder Halogen, $R^{12}$ Wasserstoff oder Alkyl mit 1 bis 8 C-Atomen, $R^{13}$ und $R^{14}$ unabhängig voneinander Wasserstoff, Halogen oder Alkyl mit 1 bis 4 C-Atomen bedeuten oder gemeinsam ein Strukturelement der Formeln a bis d

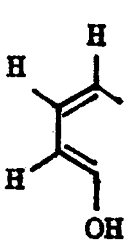 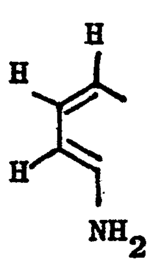 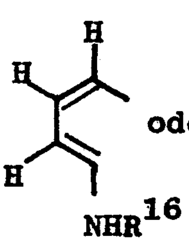 oder 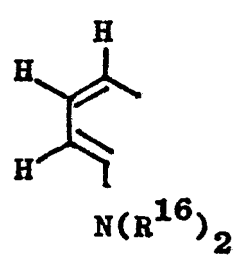

(a) (b) (c) (d)

bilden, $R^{15}$ Alkoxy mit 1 bis 4 C-Atomen, Amino, $-NHR^{16}$, $-N(R^{16})_2$, -X-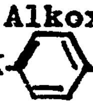-OH und, sofern $R^{13}$ und $R^{14}$ gemeinsam ein

Strukturelement der Formel a bis d bilden, auch Wasserstoff und, sofern $R^{13}$ und/oder $R^{14}$ ungleich Wasserstoff sind, auch Hydroxy, $R^{16}$ Alkyl mit 1 bis 4 C-Atomen und X -S-, $-SO_2-$, -NH- oder eine direkte Bindung bedeuten.

Es wurde nun gefunden, daß es gelingt, die Palette der Schwefelfarbstoffe um weitere sehr ansprechende und gesuchte grüne, olive und blau und graue Nuancen zu bereichern und Schwefelfarbstoffe mit für diese Klasse ausgezeichneten Echtheiten und sehr vorteilhaften Applikationsmöglichkeiten herzustellen, wenn man neue Leuco-indophenole bzw. -indamine der Formel I

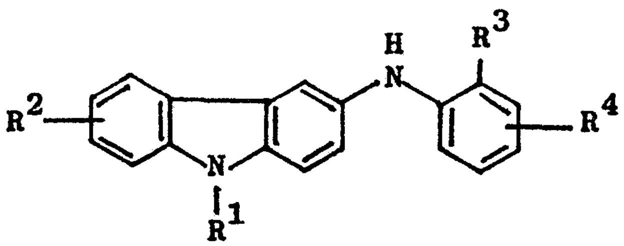

(I)

wobei $R^1$ Wasserstoff oder Alkyl mit 1 bis 8 C-Atomen, $R^2$ Wasserstoff oder Halogen, $R^3$ OH, $NH_2$ oder NHCO-Alkyl mit 1 bis 4 C-Atomen im Alkylrest und $R^4$ Wasserstoff, Halogen oder Alkyl mit 1 bis 4 C-Atomen bedeuten, nach an sich bekannten Verfahren, vorzugsweise nach dem Verfahren der Kochschmelze, schwefelt. Die für $R^1$ und $R^4$ stehenden Alkylreste sowie die Alkylreste in der für $R^3$ stehenden Gruppe -NHCO-Alkyl können geradkettig oder verzweigt sein. Als für $R^2$ und $R^4$ stehendes Halogen kommen normalerweise Fluor oder Brom, insbesondere aber Chlor, in Betracht. Von den Verbindungen der Formel I und von den aus ihnen herstellbaren Schwefelfarbstoffen sind diejenigen bevorzugt, bei denen $R^1$ Wasserstoff oder Alkyl mit 1 bis 8 C-Atomen, insbesondere Alkyl mit 1 bis 4 C-Atomen, $R^2$ Wasserstoff, $R^3$ OH oder $NH_2$ und $R^4$ Wasserstoff oder Alkyl mit 1 bis 4 C-Atomen bedeuten.

Die Verbindungen der Formel I können auch dann zu erfindungsgemäßen Schwefelfarbstoffen geschwefelt werden, wenn sie die bei der Herstellung anfallenden Isomeren, bei denen die Phenylaminogruppe in der 1-Stellung des Carbazolsystems gebunden ist, in den technisch üblichen Anteilen von bis zu ca. 30 Gew. % enthalten.

Das Schwefeln der Verbindungen I in der Kochschmelze erfolgt in der an sich bekannten Weise durch Reaktion mit Schwefel und/oder Alkalipolysulfid in wäßrigem Medium oder einem organischen Lösungsmittel bei erhöhter Temperatur, wie es z.B. bei O. Lange "Die Schwefelfarbstoffe, ihre Herstellung und Verwendung" (1912), Seiten 208 bis 220 sowie 223 bis 225, oder in Venkataraman "The Chemistry of Synthetic Dyes", Band 2 (1952), Seite 1062 ff., und Seite 1103 ff., sowie Band 7 (1974), Seite 24 ff., Academic Press, New York, San Francisco, London, beschrieben ist. Besonders geeignet sind Kochschmelzen, die unter Verwendung von Alkalipolysulfid arbeiten, wobei pro Mol des zu schwefelnden Produkts 0,1 bis 10 Mole, vorzugsweise 0,5 bis 4 Mole Natriumsulfid und 1 bis 30 Mole, vorzugsweise 2 bis 20 Mole Schwefel eingesetzt werden. Sie werden in den für Schwefelschmelzen üblichen Lösungsmitteln durchgeführt. Als Lösungsmittel, in denen zweckmäßigerweise gearbeitet werden kann, sind Alkanole oder Cycloalkanole mit 1 bis 7 C-Atomen, wie z.B. Methanol, Ethanol, Propanol, Isopropanol, n-Butanol, Isobutanol, Amylalkohol, Cyclohexanol, Methylcyclohexanol und Monoalkylether, insbesondere Monoalkylether von Ethylenglykol und Di-ethylenglykol, wie z.B. Ethylenglykolmonomethylether, -mono-ethylether, -mono-propylether, Diethylenglykol-mono-methylether, -monoethylether und -mono-propylether geeignet.

Die Schwefelung der Verbindungen der Formel I kann unter Ausschluß von Wasser oder in rein wäßrigem Medium erfolgen, wird aber normalerweise in wasserhaltigen Lösungsmitteln mit einem Wassergehalt von 5 bis 70 % durchgeführt. Im Falle der mit Wasser mischbaren Lösungsmittel arbeitet man vorzugsweise unter Verwendung der üblichen hydrotropen Verbindungen, z.B. des Natriumsalzes der Xylolsulfonsäure.

Die Kochschmelze erfolgt bei Temperaturen von 80 bis 300°C, vorzugsweise 110 bis 240°C, insbesondere im Bereich von 120 bis 170°C, und zweckmäßigerweise bei einer Reaktionsdauer von 1 bis 100, vorzugsweise von 20 bis 80 Stunden.

Unter Inkaufnahme der bekannten Nachteile der sogenannten "Backschmelzen" oder "Schwefelschmelzen" (vgl. O. Lange "Die Schwefelfarbstoffe" (1912) Seiten 221 und 222) können die Verbindungen der Formel I auch nach diesem Verfahren zu Schwefelfarbstoffen geschwefelt werden.

Bei der Herstellung der erfindungsgemäßen Schwefelfarbstoffe durch Backschmelze werden diese mit der 7- bis 30fachen Molmenge Schwefel und der 2- bis 6-fachen Molmenge Natriumsulfid 1 bis 20 Stunden auf 200 bis 300°C erhitzt. Auch hierbei kann zunächst im Lösungsmittel, vorzugsweise in Wasser, gearbeitet werden, um eine gute Homogenität des Reaktionsansatzes zu gewährleisten. Das Lösungsmittel wird dann abgedampft und die lösungsmittelfreie Schmelze zweckmäßigerweise in Schutzgasatmosphäre, z.B. unter Stickstoff, auf Temperaturen von 180 bis 300°C, vorzugsweise von 200 bis 250°C, erhitzt. Die Reaktionsdauer beträgt normalerweise 1 bis 5 Tage, vorzugsweise 1 bis 3 Tage.

Die nach den an sich bekannten Verfahren herstellbaren erfindungsgemäßen Schwefelfarbstoffe können auf übliche Weise isoliert und danach in die verschiedenen Handelsformen gebracht werden. Wurde die Schwefelung in einem mit Wasser mischbaren Lösungsmittel in Gegenwart von hydrotropen Verbindungen ausgeführt, so kann die Schmelze auch ohne Isolierung des Farbstoffs direkt zu flüssigen gebrauchsfertigen Farbstoffen weiterverarbeitet werden.

Die erfindungsgemäßen Schwefelfarbstoffe lassen sich durch Reduktionsmittel, wie sie zur Reduktion von Schwefelfarbstoffen üblich sind, sehr gut reduzieren und ergeben so klare wäßrige Lösungen. Sie können zum Färben von vorzugsweise pflanzlichen Fasern (Zellulose) nach den üblichen, für das Färben mit Schwefel- oder Schwefelküpenfarbstoffen bekannten Verfahren eingesetzt werden. Hierzu werden sie mit Reduktionsmitteln, vorzugsweise Natriumdithionit, Natriumsulfid oder Natriumhydrogensulfid, möglicherweise aber auch mit Natriumformaldehyd-sulfoxylat, Glukose oder organischen Mercaptoverbindungen, wie z.B. Thioglycerin oder Thioglycolsäure, in

die lösliche Leucoform überführt, die auf die Fasern aufzieht. Die in flüssiger, gebrauchsfertiger Form vorliegenden Farbstoffe enthalten die lösliche Leucoform und Reduktionsmittel und können auch ohne weiteren Zusatz von Reduktionsmitteln zum Färben der genannten Fasern verwendet werden.

Nach dem Aufziehen der Farbstoffe auf die Fasern wird die Leucoform der erfindungsgemäßen Schwefelfarbstoffe in üblicher Weise, beispielsweise durch "Verhängen" der Färbungen an der Luft oder Oxidation mit Oxidationsmitteln, wie beispielsweise Wasserstoffperoxid, Alkalibichromat, Alkalichlorit oder Alkalijodat, wieder in die unlösliche Form des Schwefelfarbstoffs überführt. Die erfindungsgemäß herstellbaren Schwefelfarbstoffe färben die Zellulose in vollen grünen blaugrünen, oliven und blauen und grauen Tönen und stellen damit eine überaus wertvolle Ergänzung der in den coloristischen Eigenschaften vergleichbaren Carbazolschwefelfarbstoffen C.I. Sulfur Blue 42 und 43 dar. Ein weiterer Vorteil der erfindungsgemäßen Farbstoffe ist ihre Stabilität gegen Überreduktion bei der Einwirkung von Alkalidithionit oder Alkali-Formaldehyd-sulfoxylat sowie ihre einfach technische Herstellbarkeit. Auch Polyamid läßt sich mit den erfindungsgemäßen Schwefelfarbstoffen in üblicher Weise färben. Man erhält je nach Reduktionsmittel und eingesetzter Farbstoffmenge grüne bis schwarze Farbtöne.

Die zur Herstellung der erfindungsgemäßen neuen Schwefelfarbstoffe einzusetzenden Leuco-indophenole und -indamine der Formel I sind ebenfalls neu. Sie werden erhalten, indem ein 3-Aminocarbazol der Formel V

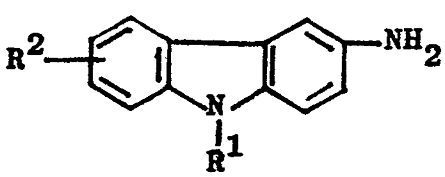

(V)

worin $R^1$ und $R^2$ die bereits angegebenen Bedeutungen haben, und/oder technisches Isomerengemisch mit einem Phenol oder Aminobenzol der Formel VI

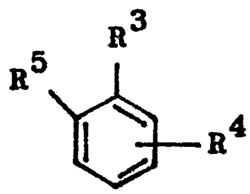

(VI)

worin $R^3$ und $R^4$ die bereits angegebenen Bedeutungen besitzen und $R^5$ OH oder $NH_2$ bedeutet, unter Ausschluß von Sauerstoff auf Temperaturen von 150 bis 300°C, zweckmäßigerweise von 180 bis 280°C, vorzugsweise von 220 bis 260°C, erhitzt wird, wobei pro Mol 3-Aminocarbazol der Formel V mindestens ein Mol der Verbindung VI eingesetzt wird.

Unter dem technischen Isomerengemisch des 3-Aminocarbazols der Formel V ist ein Produkt zu verstehen, das die bei der Herstellung des 3-Aminocarbazols im technischen Maßstab entstehenden isomeren Aminocarbazole enthält. Derartige rohe, technische Produkte enthalten neben dem Hauptprodukt, dem 3-Aminocarbazol, vorwiegend die entsprechenden 1-Amino-carbazole. Demgemäß enthalten die erfindungsgemäßen Leuco-carbazolindophenole und-indamine der Formel I, die, ausgehend von technischen, rohen 3-Aminocarbazolen, erhalten werden, in der Regel auch Anteile der isomeren Verbindungen, welche die Phenylaminogruppe in der 1-Stellung des Carbazolgerüstes enthalten.

Bei der Umsetzung des 3-Aminocarbazols der Formel V oder des entsprechenden Isomerengemischs mit der Verbindung VI werden die Komponenten im einfachsten Fall zusammengeschmolzen, gegebenenfalls mit der Verbindung der Formel VI im stöchiometrischen Überschuß und so lange auf der oben genannten Kondensationstemperatur, vorzugsweise bei 220 bis 260°C, gehalten, bis kein Reaktionswasser oder Ammoniak aus dem Ansatz mehr abdestilliert. Je nach der gewählten Reaktionstemperatur ist die Reaktion in der Regel nach 2 bis 10 Stunden praktisch abgeschlossen. Die Aufarbeitung des Reaktionsgemischs erfolgt im einfachsten Fall durch Abdestillieren eines eventuellen Überschusses der Verbindungen der Formel VI unter vermindertem Druck und anschließendes Ablassen der Schmelze aus dem Reaktionsgefäß und nachfolgendes Zerkleinern in einer geeigneten Mühle. Eine andere bequeme Möglichkeit, überschüssige Verbindungen der Formel VI, die eine -OH-Gruppe enthalten, aus dem Reaktionsprodukt zu entfernen, ist die Behandlung des Produkts mit wäßrigen Alkalihydroxidlösungen.

In vielen Fällen ist es möglich, zum Zwecke der Reinigung die entstandene Verbindung der Formel I zu destillieren, was direkt aus dem Reaktionsgefäß heraus geschehen kann. Diese Destillation wird zweckmäßigerweise unter vermindertem Druck vorgenommen. Es war außerordentlich überraschend, daß Verbindungen der Formel I sich durch eine derartige Herstellung und Destillation auf einfachstem Wege reinigen lassen. Selbst bei Sumpftemperaturen von 300°C und mehr findet praktisch keine Zersetzung statt.

Die Kondensationsreaktion zwischen den Aminocarbazolen der Formel V und der Verbindung der Formel VI kann durch einen Zusatz einer katalytischen Menge von Jod deutlich beschleunigt werden und führt zusätzlich zu reineren Produkten. Als katalytische Mengen kommen normalerweise Zusätze von 0,1 bis 4, vorzugsweise 0,1 bis 1 Gew.%, bezogen auf das eingesetzte Carbazolderivat der allgemeinen Formel V, in Betracht. Da der Jodzusatz weder bei der Aufarbeitung der erfindungsgemäß hergestellten Verbindungen der Formel I noch bei deren Weiterverarbeitung stört, ist die Durchführung des erfindungsgemäßen Verfahrens unter Zusatz von katalytischen Mengen Jod bevorzugt.

Die erfindungsgemäße Kondensation der Verbindungen der Formeln V und VI kann auch in Gegenwart eines inerten organischen Lösungsmittels durchgeführt werden. Zweckmäßigerweise werden solche Lösungsmittel verwendet, deren Siedepunkt oberhalb der Kondensationstemperatur, d.h. insbesondere oberhalb von 150 bis 260°C liegt. Sofern aufgrund besonderer Überlegungen Lösungsmittel eingesetzt werden sollen, deren Siedepunkt tiefer als 150°C liegt, ist zweckmäßigerweise in einem druckfest verschlossenen Gefäß (Autoklav) zu arbeiten. Organische Lösungsmittel, die für die Durchführung des erfindungsgemäßen

Verfahrens geeignet sind, sind z.B. Erdölfraktionen mit einem Siedepunkt über 150°C, cyclische Kohlenwasserstoffe, wie beispielsweise Decalin, oder aromatische Lösungsmittel, insbesondere das technische Gemisch aus Monochlor- und Dichlorbenzol. Insbesondere wenn die hergestellten Verbindungen der Formel I anschließend in einer sogenannten Kochschmelze, d.h. einer Lösung von Natriumpolysulfiden, in Schwefelfarbstoffe überführt werden, kann die erfindungsgemäße Kondensation der Verbindung der Formel VI mit einem Carbazolderivat der allgemeinen Formel V auch in Gegenwart eines Lösungsmittels der allgemeinen Formel VII oder VIII

$R^{20}O-(CH_2CH_2O)_n-R^{20}$ (VII) $R^{20}O-(CH_2CH_2O)_n-H$ (VIII)

worin $R^{20}$ = $-CH_3$, $-C_2H_5$, $-C_3H_7$ und n = 1, 2, 3 oder 4 bedeuten, oder in einem höheren Alkohol oder Alkanolether durchgeführt werden. Geeignete Lösungsmittel der allgemeinen Formel VII und VIII sind z.B. Diglykoldimethylether, Diglykoldiethylether, Glykoldiethylether, Ethylenglykol-mono-methylether, -mono-ethylether, -mono-propylether, Diethylenglykol-monomethylether, -mono-propylether. Bei Verwendung von Lösungsmitteln der allgemeinen Formel VII oder VIII oder in einem höheren Alkohol oder Alkanolether kann die rohe Reaktionsmischung des Leuco-indophenols der Formel I direkt der Schwefelung unterworfen werden.

Wenn die Herstellung der Verbindungen der allgemeinen Formel I nach dem erfindungsgemäßen Verfahren in dem zweckmäßigen Temperaturbereich von 180 bis 280°C oder in dem bevorzugten Temperaturbereich von 220 bis 260°C in Gegenwart eines inerten Lösungsmittels durchgeführt wird, so sollte dieses bei Normaldruck zweckmäßigerweise einen Siedepunkt von 190 bis 290°C bzw. 230 bis 270°C oder höher, besitzen. Zweckmäßigerweise wird ein solches inertes Lösungsmittel ausgewählt, das mit Wasser bei Normaltemperatur nicht mischbar ist. Derartige inerte Lösungsmittel wirken bei der Umsetzung des Carbazolderivats V mit Verbindungen der Formel VI, in denen $R^5$ -OH ist, als azeotrope Schleppmittel für das bei der Reaktion abgespaltene Wasser. Derartige als azeotrope Schleppmittel geeignete Lösungsmittel sind beispielsweise Decalin, hochsiedende Benzine, Dichlorbenzol, Trichlorbenzol.

Bei der Verwendung eines inerten Lösungsmittels wird dessen Menge so gering wie möglich gehalten. In der Regel ist es nicht erforderlich, mehr als 15 Gew.% des inerten Lösungsmittels, bezogen auf das Gesamtgewicht der Reaktanten, einzusetzen.

Der Ausschluß von Sauerstoff bei der Herstellung der erfindungsgemäßen Verbindungen der Formel I wird z.B. dadurch bewirkt, daß die Reaktion unter der Atmosphäre eines Inertgases, wie z.B. Stickstoff, durchgeführt wird. Insbesondere wenn kein inertes Lösungsmittel verwendet wird, ist es zweckmäßig, mindestens die 1,5fache stöchiometrische Menge, vorzugsweise die 2,5fache stöchiometrische Menge der Verbindung der Formel VI einzusetzen. Der eingesetzte Überschuß wirkt dabei als Lösungsmittel. Höhere Überschüsse als die 3fache stöchiometrische Menge brauchen normalerweise nicht eingesetzt zu werden, stören jedoch nicht. Nach beendeter Reaktion kann in den meisten Fällen die überschüssige Verbindung der Formel VI leicht, zweckmäßig unter vermindertem Druck abdestilliert und in einem weiteren Ansatz wieder verwendet werden. Auch hier empfiehlt sich bei Einsatz von Verbindungen der Formel VI, die eine OH-Gruppe aufweisen, die Extraktion mit wäßriger Alkalihydroxidlösung. Bei der Durchführung der Reaktion zwischen dem Carbazolderivat der allgemeinen Formel III und der Verbindung der Formel VI kann der Reaktionsverlauf entweder dünnschichtchromatographisch und/oder bei $R^5$ = -OH durch Bestimmung des abgespaltenen Wassers oder bei $R^5$ = $-NH_2$ durch titrimetrische Bestimmung des abgespaltenen Ammoniaks verfolgt werden.

Die erfindungsgemäße Herstellung der Verbindungen der Formel I ist technisch einfach durchzuführen. Man erhält vor allem

bei der bevorzugten Ausführung unter Zusatz von Jod ausgezeichnete Ausbeuten, insbesondere sehr gute Raum-Zeit-Ausbeuten. Es bedarf keiner umständlichen Isolierungsmaßnahmen des Reaktionsprodukts, es entstehen praktisch keine Abwässer, Abfallösungsmittel oder sonstige ökologisch bedenklichen Abfallprodukte, und es können vor allem bei der bevorzugten Ausführung unter Zusatz von Jod Produkte von hoher Reinheit erhalten werden, die zur Herstellung von Schwefelfarbstoffen ausgezeichneter und reproduzierbarer Nuance und Farbreinheit dienen können. Im Hinblick auf die unterschiedliche Weiterverarbeitung der hergestellten Indophenole und Indamine der Formel I ist die Durchführung des erfindungsgemäßen Verfahrens ohne jedes Lösungsmittel am universellsten anwendbar.

Die Verbindungen der allgemeinen Formeln V und VI sind bekannte, zum Teil großtechnisch hergestellte Produkte. Sofern sie nichthandelsüblich sind, lassen sie sich nach einfachen, bekannten Verfahren gut herstellen.

Beispiele für geeignete Aminocarbazole der Formel V sind: 3-Aminocarbazol, 3-Amino-9-ethylcarbazol, 3-Amino-9-butylcarbazol, 3-Amino-6-chlor-9-ethylcarbazol, 3-Amino-9-octylcarbazol. Beispiele für geeignete Hydroxy- bzw. Aminoverbindungen der Formel VI sind: Brenzcatechin, 4-Chlor-Brenzcatechin, 4-Methylbrenzcatechin, ortho-Phenylendiamin, 4-Chlorortho-phenylendiamin, 4-Methyl-ortho-phenylendiamin, N-Acetylortho-phenylendiamin, ortho-Aminophenol etc.

Die folgenden Ausführungsbeispiele veranschaulichen die Herstellung und Verwendung der erfindungsgemäßen Schwefelfarbstoffe und die Herstellung der erfindungsgemäßen Zwischenprodukte.

<u>Beispiel 1</u>

a) Eine Mischung von 200 ml Diethylenglycol-mono-ethylether, 90 g Schwefel, 71 g Natriumsulfid konz. (60 %ig), 50 g xylolsulfonsaures Natrium und 50 g des Leuco-carbazolindophenols der Formel

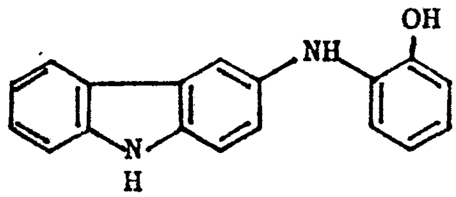

wird auf 143°C erwärmt und bei dieser Temperatur 48 Stunden am Rückflußkühler gerührt. Dann wird die heiße Schmelze in 2 l 60°C warmes Wasser eingerührt, die erhaltene Farbstoffsuspension mit 50 g Natriumhydrogensulfit versetzt, 2 Stunden bei 70 bis 80°C gerührt und abgesaugt. Der Farbstoff wird auf dem Filter mit Wasser neutral gewaschen und getrocknet. Die Ausbeute beträgt 62 g. Aus der Dithionitküpe färbt der Farbstoff Baumwolle in einem blaustichig grauen Farbton. Die Färbung hat sehr gute Naßechtheiten und gute Lichtechtheit.

b) Das im vorstehenden Absatz a) eingesetzte Leuco-indophenol 3-(2-Hydroxyphenylamino)-carbazol wird wie folgt hergestellt:

640 g Brenzcatechin werden unter Stickstoff geschmolzen, 6 g Jod zugefügt und innerhalb von 2 Stunden 364 g 3-Aminocarbazol eingetragen. Es wird unter Rühren bei ca. 250°C Innentemperatur das entstehende Reaktionswasser abdestilliert. Nach ca. 12 Stunden Reaktionszeit ist alles Aminocarbazol umgesetzt. (Das Fortschreiten der Reaktion wird dünnschichtchromatographisch mit Toluol/Essigester 1 : 6 als Fließmittel verfolgt.) Nun wird das überschüssige Brenzcatechin im Wasserstrahlvakuum abdestilliert.

Man erhält 523 g = 95,4 % der Theorie 3-(2-Hydroxyphenylamino)-carbazol vom Fp: 95 bis 100°C.

<u>Beispiel 2</u>

a) Eine Mischung von 200 ml Diethylenglycol-mono-ethylether, 90 g Schwefel, 71 g Natriumsulfid konz. (60 %ig) und 50 g des Leuco-Carbazolindophenols der Formel

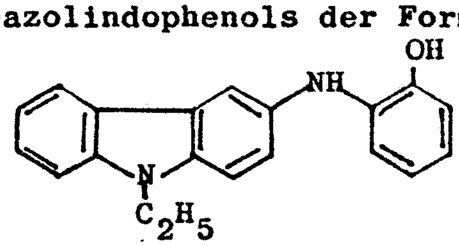

wird auf eine Temperatur von 137 bis 139°C erwärmt. Bei dieser Temperatur wird der Ansatz am Rückflußkühler 42 Stunden gerührt. Nach Ablauf dieser Zeit wird die heiße Schmelze in 2 l 60°C warmes Wasser eingerührt.

Die erhaltene Farbstoffsuspension wird mit 100 g Natriumhydrogensulfit versetzt und 2 Stunden bei 70 bis 80°C gerührt. Anschließend wird der Farbstoff abgesaugt, mit Wasser neutral gewaschen und getrocknet. Ausbeute 105 g Farbstoff.

Der Farbstoff färbt auf Baumwolle aus der Dithionitküpe ein grünstichiges Grau mit sehr guten Naßechtheiten und guter Lichtechtheit.

b) Das im vorstehenden Absatz eingesetzte Leuco-carbazolindophenol 3-(2-Hydroxyphenylamino)-9-ethylcarbazol wird wie folgt hergestellt:

250 g Brenzcatechin und 2 g Jod werden mit 210 g N-Ethyl-3-aminocarbazol, wie in Beispiel 1a angegeben, umgesetzt.
Nach dem Abdestillieren des überschüssigen Brenzcatechins erhält man 280 g = 93 % der Theorie 3-(2-Hydroxyphenylamino)-9-ethylcarbazol vom Fp. 95 bis 105°C.

Beispiel 3

a) Eine Mischung von 200 ml Diäthylenglycol-mono-ethylether, 90 g Schwefel, 71 g Natriumsulfid konz. (60 %ig), 50 g xylolsulfonsaures Natrium und 50 g des Leuco-carbazolindamins der Formel

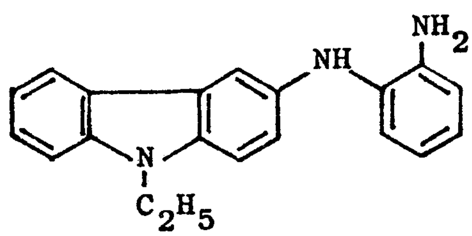

wird auf 138°C erwärmt und bei dieser Temperatur 42 Stunden am Rückflußkühler gerührt. Dann wird die heiße Schmelze in 1 l 60°C warmes Wasser eingerührt, die erhaltene Farbstoffsuspension mit 50 g Natriumhydrogensulfit versetzt, 2 Stunden bei 70 bis 80°C gerührt und abgesaugt. Der Farbstoff wird

auf dem Filter mit Wasser neutral gewaschen und getrocknet. Die Ausbeute beträgt 84 g. Aus der Dithionitküpe färbt der Farbstoff Baumwolle in einem vollen braunen Farbton. Die Färbung hat sehr gute Naßechtheiten und gute Lichtechtheit.

b) Das im vorstehenden Absatz eingesetzte Leuco-carbazolindamin wird wie folgt hergestellt:

250 g ortho-Phenylendiamin, 3 g Jod und 210 g N-Ethyl-3-aminocarbazol werden wie in Beispiel 1a umgesetzt (anstelle von Reaktionswasser entsteht hier Ammoniak). Nach dem Abdestillieren des überschüssigen ortho-Phenylendiamins werden 290 g = 95 % der Theorie 3-(2-Aminophenylamino)-9-ethylcarbazol vom Fp. ca. 100°C ($Kp_{1,3\ mbar}$ 260°C) erhalten.

Beispiel 4

Entsprechend den Beispielen 1a bis 3a kann N-n-Octyl-3-aminocarbazol und 1,2-Diamino-4-methylbenzol zu einem Leuco-carbazolindamin umgesetzt werden, das beim Schwefeln entsprechend den Beispielen 1a bis 3a ebenfalls einen wertvollen Schwefelfarbstoff liefert.

Beispiel 5

Entsprechend dem Beispiel 1a kann 3-Aminocarbazol und o-Amino-phenol unter Abspaltung von Ammoniak zu dem 3-(2-Hydroxyphenylamino)-carbazol umgesetzt werden.

0043537

PATENTANSPRÜCHE

1. Schwefelfarbstoffe, herstellbar durch Schwefeln von Leuco-indophenolen bzw. -indaminen der Formel I

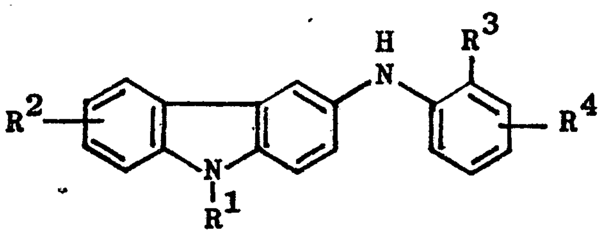

(I)

wobei $R^1$ Wasserstoff oder Alkyl mit 1 bis 8 C-Atomen, $R^2$ Wasserstoff oder Halogen, $R^3$ OH, $NH_2$ oder NHCO-Alkyl mit 1 bis 4 C-Atomen im Alkylrest und $R^4$ Wasserstoff, Halogen oder Alkyl mit 1 bis 4 C-Atomen bedeuten, nach an sich bekannten Verfahren.

2. Schwefelfarbstoffe gemäß Anspruch 1, wobei in dem Leuco-indophenol bzw. -indamin der Formel I $R^1$ Wasserstoff oder Alkyl mit 1 bis 8 C-Atomen, insbesondere mit 1 bis 4 C-Atomen, $R^2$ Wasserstoff, $R^3$ OH oder $NH_2$ und $R^4$ Wasserstoff bedeuten.

3. Schwefelfarbstoffe gemäß den Ansprüchen 1 oder 2, wobei die Verbindungen der Formel I nach dem an sich bekannten Verfahren der Kochschmelze geschwefelt werden.

4. Verfahren zur Herstellung von Schwefelfarbstoffen durch Umsetzung von Ausgangsmaterialien mit Schwefel und Alkalisulfid oder Alkalipolysulfid nach dem Verfahren der Kochschmelze oder der Backschmelze, dadurch gekennzeichnet, daß als Ausgangsmaterialien Leuco-indophenole bzw. -indamine der Formel I

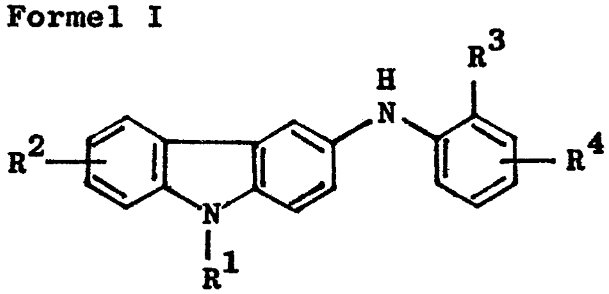

(I)

wobei $R^1$ Wasserstoff oder Alkyl mit 1 bis 8 C-Atomen, $R^2$ Wasserstoff oder Halogen, $R^3$ OH, $NH_2$ oder NHCO-Alkyl mit 1 bis 4 C-Atomen im Alkylrest und $R^4$ Wasserstoff, Halogen oder Alkyl mit 1 bis 4 C-Atomen bedeuten, eingesetzt werden.

5. Verfahren zur Herstellung von Schwefelfarbstoffen gemäß Anspruch 4, dadurch gekennzeichnet, daß Indophenole oder Indamine der Formel I, wobei $R^1$ Wasserstoff oder Alkyl mit 1 bis 8 C-Atomen, insbesondere mit 1 bis 4 C-Atomen, $R^2$ Wasserstoff, $R^3$ OH oder $NH_2$ und $R^4$ Wasserstoff bedeuten, in der Kochschmelze geschwefelt werden.

6. Leuco-indophenol bzw. -indamin der Formel I

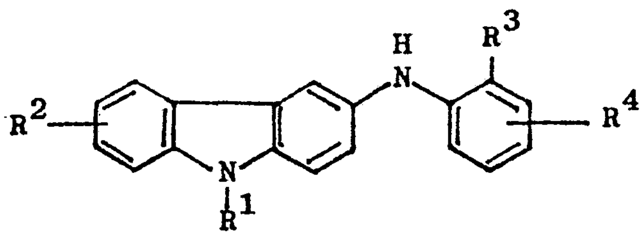

(I)

wobei $R^1$ Wasserstoff oder Alkyl mit 1 bis 8 C-Atomen, $R^2$ Wasserstoff oder Halogen, $R^3$ OH, $NH_2$ oder NHCO-Alkyl mit 1 bis 4 C-Atomen im Alkylrest und $R^4$ Wasserstoff, Halogen oder Alkyl mit 1 bis 4 C-Atomen bedeuten,

7. Leuco-indophenol bzw. -indamin nach Anspruch 6, dadurch gekennzeichnet, daß $R^1$ Wasserstoff oder Alkyl mit 1 bis 8 C-Atomen, insbesondere mit 1 bis 4 C-Atomen, $R^2$ Wasserstoff, $R^3$ OH oder $NH_2$ und $R^4$ Wasserstoff bedeuten.

8. Verfahren zur Herstellung von Leuco-indophenolen bzw. -indaminen der Ansprüche 6 oder 7, dadurch gekennzeichnet, daß ein 3-Aminocarbazol der Formel V

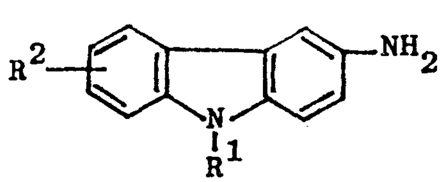

(V)

oder sein technisches Isomerengemisch mit einer Verbindung der Formel VI

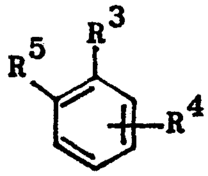

(VI)

wobei $R^1$, $R^2$, $R^3$ und $R^4$ die in den Ansprüchen 6 oder 7 angegebenen Bedeutungen besitzen und $R^5$ OH oder $NH_2$ bedeutet, unter Ausschluß von Sauerstoff auf Temperaturen von 150 bis 300°, vorzugsweise 220 bis 260°C, erhitzt wird.

9. Verfahren nach Anspruch 8, dadurch gekennzeichnet, daß die Reaktion in Gegenwart katalytischer Mengen Jod durchgeführt wird.

10. Verwendung der Schwefelfarbstoffe der Ansprüche 1 bis 3 zum Färben von Zellulose und Polyamid.

Europäisches Patentamt

**EUROPÄISCHER RECHERCHENBERICHT**

0043537

Nummer der Anmeldung

EP 81 10 5054

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | FR - A - 633 473 (SOC. IND. CHIM. A BALE )<br>* Zusammenfassung *<br>-- | 1 |
| A | GB - A - 15 949/1914 (O. IMRAY)<br>* Ansprüche 1,2 *<br>-- | 1 |
| PA | EP - A - 0 014 261 (CASSELLA)<br>* Ansprüche 1-10 * | 1 |
| D | & DE - A - 2 900 441<br>---- | |

KLASSIFIKATION DER ANMELDUNG (Int. Cl.3)

C 09 B 49/10
53/02
C 07 D 209/88
D 06 P 1/30

RECHERCHIERTE SACHGEBIETE (Int. Cl.3)

C 09 B 49/10
49/00
53/02
53/00
C 07 D 209/88
C 09 B 49/12

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 20-10-1981 | DELANGHE |